# EUROPEAN PATENT APPLICATION

(11) **EP 4 537 870 A1**
(43) Date of publication of application: **16.04.2025**
(21) Application number: 23203394.4
(22) Date of filing: 13.10.2023
(51) Int. Cl.: A61M 5/00

(54) **MEDICAL CONTAINER HOLDING DEVICE WITH RIGIDITY-ENHANCING RAISED PLATEAU**

(71) Applicant: Becton, Dickinson and Company, Franklin Lakes, NJ 07417-1880 (US)
(72) Inventor: RIVE, Adrien, 38170 Seyssinet-Pariset (FR); REMPFER, Simon, 38260 St Hilaire de la Cote (FR); VONIEZ, Jimmy, 38000 Grenoble (FR); KERGUELEN, Aude, 38000 Grenoble (FR); BARCELO, Aurélie, 38400 Saint-Martin-d'Heres (FR)
(74) Representative: Germain Maureau

(57) **Abstract**

A holding device for supporting medical containers includes a supporting plate having first and second surfaces, a peripheral edge between the first and second surfaces, and a plurality of openings through the supporting plate configured to receive the medical containers. The supporting plate further includes a peripheral lower portion extending inward from the peripheral edge, a pair of cutouts formed in opposing sides of the peripheral edge and in the peripheral lower portion, one or more raised plateaus vertically offset from the peripheral lower portion and enclosed thereby, and connecting walls extending between the peripheral lower portion and the raised plateaus, with the raised plateaus include a pair of C-shaped plateaus positioned, respectively, about the pair of cutouts. A plurality of chimneys protrude from the supporting plate and enclose the plurality of openings, such that the chimneys guide insertion of the medical containers into the openings.

## Description

### BACKGROUND OF THE INVENTION

### Field of the Invention

The present disclosure relates generally to holding devices for holding medical containers, such as cartridges, barrels, prefilled-syringes, stoppers, and/or pre-fillable syringes, in an upright position and, in particular, to plate-type holding devices including a rigidity-enhancing raised plateau, with the holding devices configured to be retained within containers or tubs for containing the medical containers.

### Description of Related Art

Medical containers, such as cartridges, barrels, prefilled-syringes, stoppers, and/or pre-fillable syringes, often need to be transported from one site to another site during or after manufacturing. For example, medical containers may be manufactured at a first site and then transported to a second site, where the medical containers are filled with a medical fluid. As used herein, a "medical fluid" can refer to a medication or another therapeutic agent used for treatment of chronic or acute conditions, as are known in the art. Exemplary therapeutic agents can include, for example, drugs, chemicals, biological, or biochemical substances that, when delivered in a therapeutically effective amount to the patient, achieve a desired therapeutic effect. In other examples, the medical containers can be manufactured and filled at a same first site and then transported to a second site for storage.

During transport, the medical containers can be held by a holding device, such as a tray, plate, and/or nest. A nest can be a plate-shaped tray configured to support multiple medical containers in an upright orientation. Nests can comprise multiple "chimneys" aligned according to predetermined rows or a predetermined pattern, with each chimney defining a receptacle or opening configured to receive one medical container. When inserted into and through the receptacle, the medical container is held in the upright orientation in a direction substantially orthogonal to the nest. The nest is usually placed inside a box-shaped tub with an open top, which can be sealed by a sealing cover. Removal of the medical containers from this tub can require peeling off the sealing cover, removing the nest holding the medical containers from the tub, and removing the medical containers from the nest by axially sliding the medical containers relative to the nest. Exemplary nests and tubs configured to contain medical containers are disclosed, for example, in: U.S. Patent No. 10,023,358 ("the '358 patent"), entitled "Packaging for containers;" U.S. Patent No. 10, 143,793 ("the '793 patent"), entitled "Tray for positioning elongated objects, in particular syringe bodies or syringes;" and U.S. Patent Appl. Pub. No. 2021/0236715A1 ("the '715 publication"), entitled "Holding Device Configured to Support a Plurality of Medical Containers Such as Syringes," which are incorporated herein by reference in their entireties.

In many cases, the medical containers are filled with the medical fluid using an automated filling machine before the medical containers are removed from the nest and individually packaged. The nests can deform during filling due to forces exerted on the medical containers and/or nest by the automated filing machine. For example, when handled by the filling machine, the nests may bend or warp due to the weight of multiple medical containers held by the nest. As a result of deformation of the nest or trays, medical containers supported by the deformed trays may not be retained in the upright orientation, which may lead to some inaccuracy during the filling process and/or during a stoppering process. Deformation of the nests or trays may also cause problems during conveying of the filled nests or trays to different locations.

For these reasons, there is a need for nest and tray designs with increased stiffness in order to reduce deflection of the nest or tray during transport and filling. Avoiding deformation can improve accuracy of the filling process by avoiding misalignment between the medical containers contained by the tray or nest and the automated filling machines. The holding devices of the present disclosure are intended to provide such increased stiffness and to resist deflection or deformation under weight of multiple medical containers contained by the tray or nest.

### SUMMARY OF THE INVENTION

Provided herein is a holding device configured to support medical containers. The holding device includes a supporting plate comprising a first surface, a second surface, a peripheral edge between the first surface and the second surface and having a first pair of opposed sides and a second pair of opposed sides, and a plurality of openings through the supporting plate configured to receive the medical containers. The supporting plate further comprises a peripheral lower portion extending inward from the peripheral edge, a pair of cutouts formed in the second pair of opposed sides and in the peripheral lower portion, one or more raised plateaus vertically offset from the peripheral lower portion and enclosed by the peripheral lower portion, and connecting walls extending between the peripheral lower portion and the one or more raised plateaus, with the one or more raised plateaus including a pair of C-shaped plateaus positioned, respectively, about the pair of cutouts. The holding device further includes a plurality of chimneys protruding from the first surface of the supporting plate and at least partially enclosing the plurality of openings, such that the plurality of chimneys guide insertion of the medical containers into the plurality of openings.

In accordance with an aspect of the disclosure, the plurality of openings are arranged on the supporting plate as a plurality of rows aligned parallel to a longitudinal axis of the supporting plate, and wherein each adjacent pair of rows of the plurality of row are offset from each other relative to the longitudinal axis, such that the plurality of rows are staggered.

In accordance with an aspect of the disclosure, the one or more raised plateaus comprise a continuously formed plateau comprising a plurality of connected plateau regions, with the plurality of connected plateau regions comprising a center plateau having a linear configuration and being formed between two centermost rows of the plurality of rows, so as to be aligned along the longitudinal axis, a first peripheral plateau and a second peripheral plateau positioned on opposing sides of the center plateau, and the pair of C-shaped plateaus positioned on opposing ends of the center plateau.

In accordance with an aspect of the disclosure, the center plateau and the first peripheral plateau encircle a first inner lower portion of the supporting plate and the center plateau and the second peripheral plateau encircle a second inner lower portion of the supporting plate, the first inner lower portion and the second inner lower portion vertically aligned with the peripheral lower portion, and wherein the connecting walls extend between the first and second inner lower portions and the center plateau and the first and second peripheral plateaus.

In accordance with an aspect of the disclosure, the connecting walls extending between the peripheral lower portion and the first and second peripheral plateaus intersect outermost peripheral chimneys of the plurality of chimneys.

In accordance with an aspect of the disclosure, the connecting walls extending between the peripheral lower portion and the first and second peripheral plateaus are positioned outside of a receiving region comprising the plurality of chimneys, so as to not be intersected by any chimneys of the plurality of chimneys, with the first and second peripheral plateaus thus extending out past outermost peripheral chimneys of the plurality of chimneys.

In accordance with an aspect of the disclosure, the one or more raised plateaus comprise a cross-shaped plateau including a first plateau arm and a second plateau arm oriented orthogonal to each other and the pair of C-shaped plateaus positioned on opposing ends of the first plateau arm.

In accordance with an aspect of the disclosure, the supporting plate includes a peripheral wall oriented orthogonal to the supporting plate and extending up from the peripheral lower portion, wherein the peripheral wall intersects and is joined to the cross-shaped plateau.

In accordance with an aspect of the disclosure, the peripheral wall is positioned to intersect outermost peripheral chimneys of the plurality of chimneys, and wherein the peripheral wall joins to opposing ends of the second plateau arm and intersects the first plateau arm at locations thereof that are aligned with the outermost peripheral chimneys.

In accordance with an aspect of the disclosure, the peripheral wall is positioned outside of a receiving region comprising the plurality of chimneys, so as to not be intersected by any chimneys of the plurality of chimneys, and wherein the peripheral wall joins to opposing ends of the second plateau arm to the pair of C-shaped plateaus outside of the receiving region.

In accordance with an aspect of the disclosure, each of the first plateau arm and the second plateau arm includes two bump-out plateaus formed thereon that are further elevated as compared to a remainder of the first plateau arm and the second plateau arm.

In accordance with an aspect of the disclosure, the one or more raised plateaus are elevated relative to the peripheral lower portion by a height that is less than or equal to a height of the plurality of chimneys, such that the one or more raised plateaus intersect at least some chimneys of the plurality of chimneys.

In accordance with an aspect of the disclosure, the supporting plate comprises a plurality of reinforcing ribs extending between at least some of the plurality of chimneys, the reinforcing ribs connecting a respective chimney to one or more adjacent chimneys, wherein the reinforcing ribs are orthogonal to the supporting plate and extend up from a bottom end of a respective chimney to the height of the one or more raised plateaus.

In accordance with an aspect of the disclosure, the plurality of reinforcing ribs extend between adjacent chimneys encompassed by the center plateau and the pair of C-shaped plateaus, with the reinforcing ribs extending up from the bottom ends of the chimneys to an underside of the center plateau and the pair of C-shaped plateaus.

In accordance with an aspect of the disclosure, the plurality of reinforcing ribs extend between adjacent chimneys encompassed by the first plateau arm and the pair of C-shaped plateaus, with the reinforcing ribs extending up from the bottom ends of the chimneys to an underside of the first plateau arm and the pair of C-shaped plateaus.

In accordance with an aspect of the disclosure, respective connecting walls extend between the pair of C-shaped plateaus and the pair of cutouts, providing curved walls at the pair of cutouts that provide finger grips by which a user can grasp the holding device.

In accordance with an aspect of the disclosure, the connecting walls are substantially vertical, extending substantially orthogonally from the lower portion, and having a draft angle of about 0.1 degree to about 4.0 degrees.

In accordance with an aspect of the disclosure, the plurality of chimneys are configured to receive barrels of syringes or cartridges containing a fluid volume of 50 mL or less, preferably from 1-10 mL, and even more preferably from 1-3 mL.

In accordance with an aspect of the disclosure, the peripheral lower portion and the one or more raised plateaus are flat.

Also provided herein is a method of manufacture for the holding device as described herein. The method includes forming the support plate and the plurality of chimneys as a single, integral component.

### BRIEF DESCRIPTION OF THE DRAWINGS

FIG. 1A is a top perspective view of a holding device configured to support medical containers, according to an aspect of the present disclosure;
FIG. 1B is a bottom perspective view of the tray of FIG. 1A;
FIG. 1C is a top view of the tray of FIG. 1A;
FIG. 1D is a bottom view of the tray of FIG. 1A;
FIG. 1E is a cross-sectional view of the tray of FIG. 1A;
FIG. 2A is a top perspective view of a holding device configured to support medical containers, according to another aspect of the present disclosure;
FIG. 2B is a top view of the holding device of FIG. 2A;
FIG. 2C is a bottom view of the holding device of FIG. 2A;
FIG. 3A is a top perspective view of a holding device configured to support medical containers, according to another aspect of the present disclosure;
FIG. 3B is a top view of the tray of FIG. 3A;

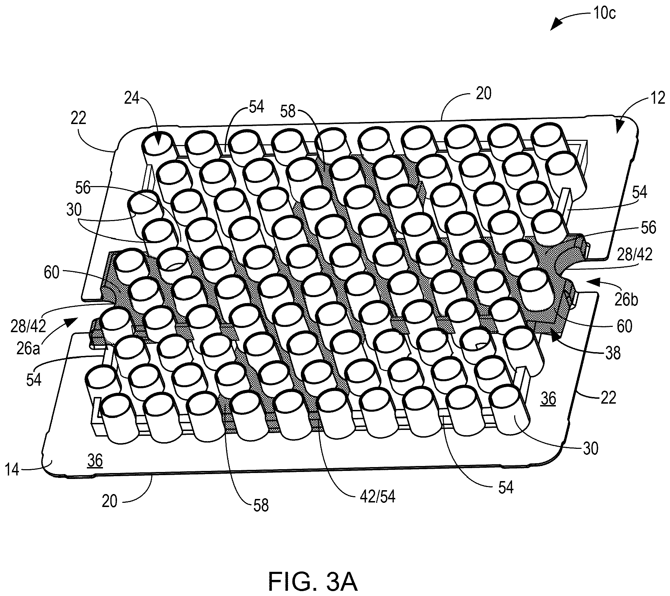

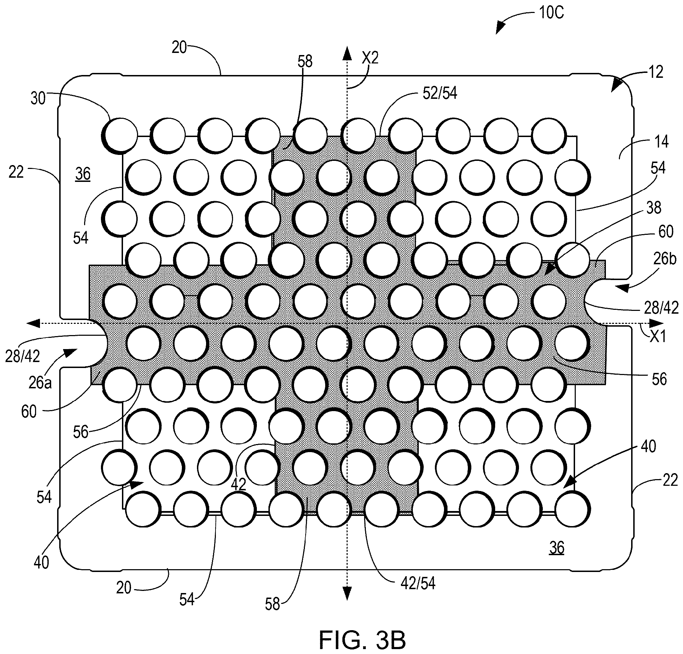

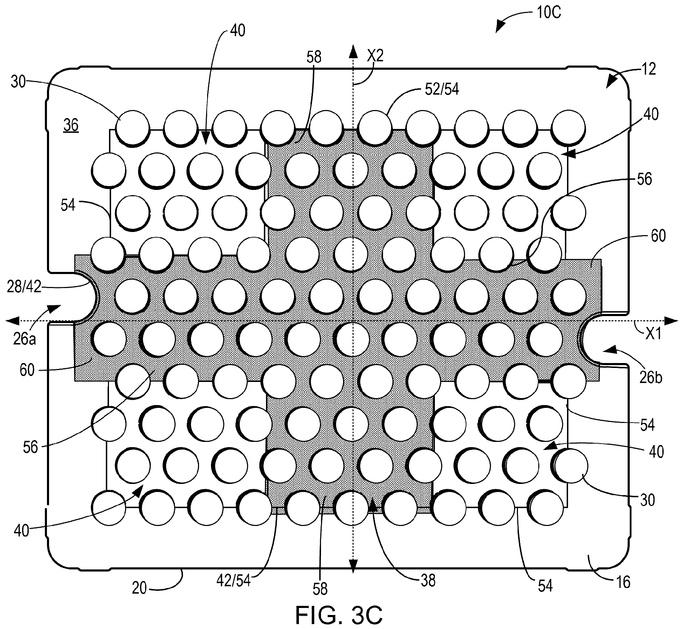

FIG. 3B is a bottom view of the tray of FIG. 3A;

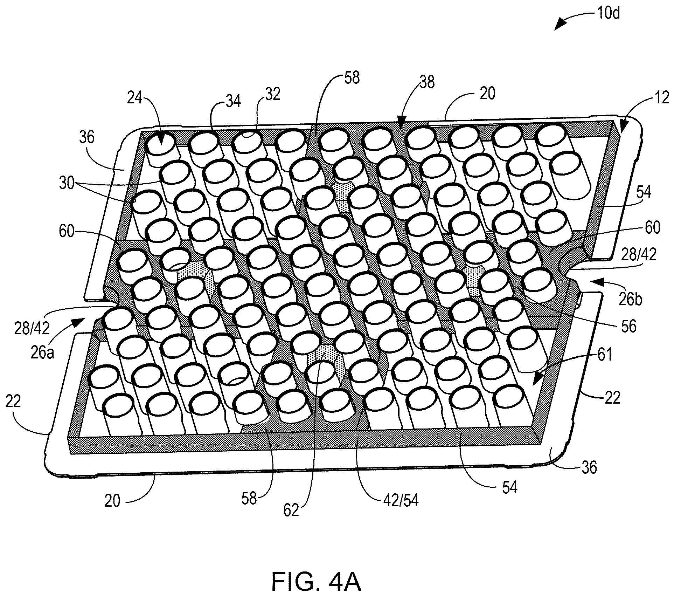

FIG. 4A is a top perspective view of a holding device configured to support medical containers, according to another aspect of the present disclosure.

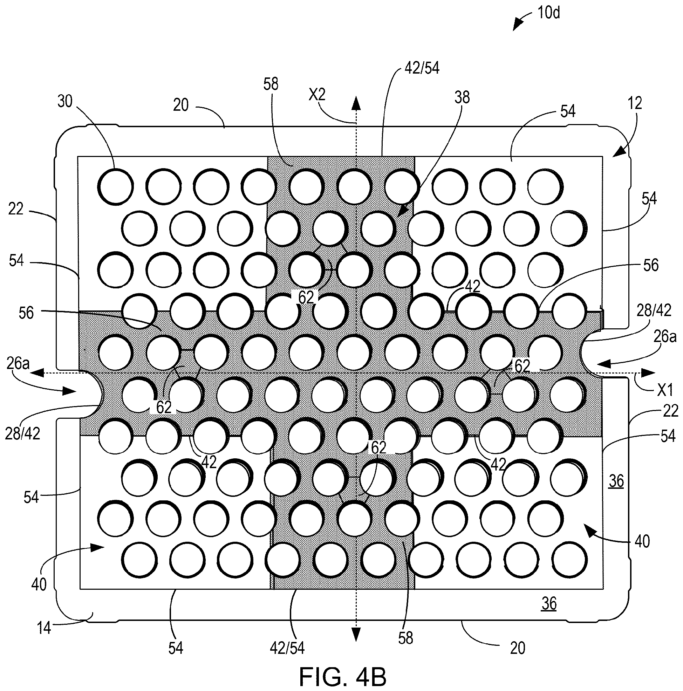

FIG. 4B is a top view of the tray of FIG. 4A; and

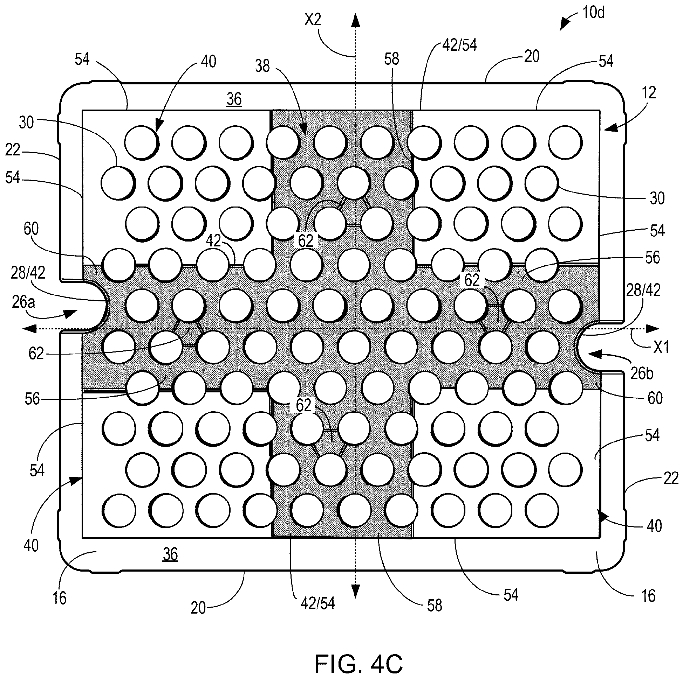

FIG. 4C is a bottom view of the tray of FIG. 4A.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", "transverse", and derivatives thereof shall relate to the invention as it is oriented in the drawing figures. However, it is to be understood that the invention may assume alternative variations and step sequences, except where expressly specified to the contrary. It is also to be understood that the specific devices and processes illustrated in the attached drawings, and described in the following specification, are simply exemplary embodiments of the invention. Hence, specific dimensions and other physical characteristics related to the embodiments disclosed herein are not to be considered as limiting.

With reference to the figures, the present disclosure is directed to holding devices 10 comprising openings or cavities for retaining multiple medical containers, such as multiple cartridges, barrels, stoppers, prefilled-syringes, and/or pre-fillable syringes, in an upright position. In particular, the holding devices 10 can be configured to concurrently support multiple medical containers in the upright position during packaging, storing, transporting, and/or handling of the multiple medical containers with an automated filling machine.

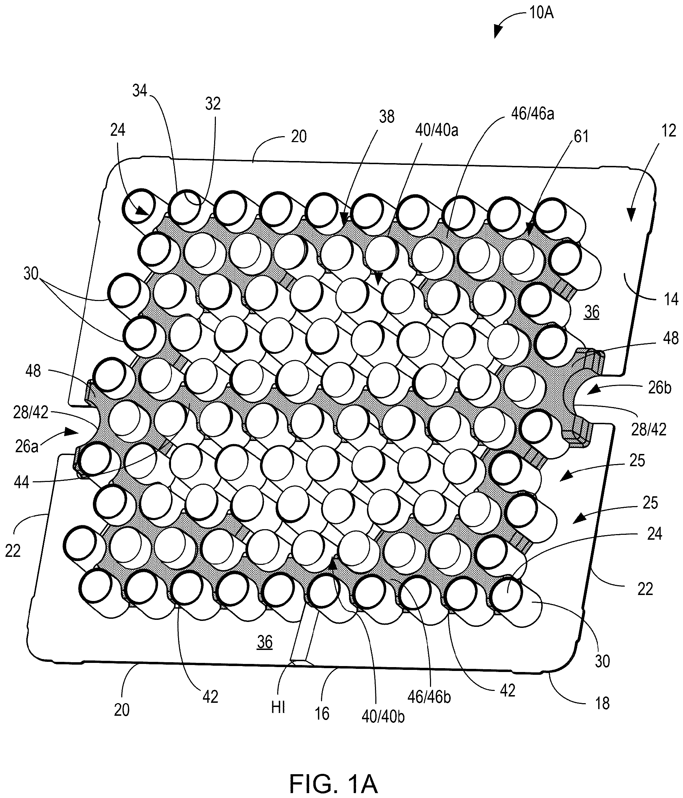

A medical container, such as a syringe, is generally an elongate structure that comprises a hollow elongated barrel defining a reservoir for containing the medical fluid, a stopper located inside the barrel, and a plunger rod to move the stopper through the barrel to expel the medical fluid through a tip, fluid port, nozzle, or needle cannula at an end of the barrel. The medical containers can also include a flange at an end of the barrel, which can provide a surface for positioning a user's fingers (e.g., the index and the middle fingers), while the plunger rod is activated with the thumb. The reservoirs of the medical containers held by the holding devices 10 of the present disclosure can contain less than about 50 mL of the medical fluid, or preferably from about 1 mL to 10 mL, and even more preferably from 1 mL to 3 mL. The holding devices 10 can be configured to contain 160 or fewer medical containers. For example, the holding device 10 can be configured to contain 100 medical containers, as shown in FIGS. 1A-1E.

According to aspects of the disclosure, the holding devices 10 may be configured as a plate-type structure that includes surfaces of different elevations in order to increase stiffness of the holding device 10 and to resist deformation of the holding device 10. In particular, the holding devices 10 can be configured to resist deformation when the holding devices 10 are used with the automated filling machine, such that downward deflection of the holding device 10 is 2.5 mm or less during filling, when the holding device 10 is substantially fully loaded with filled medical containers. Specifically, the holdings devices 10 can be configured to deflect by about 2.5 mm or less, for a holding device 10 having a thickness of approximately 1.3 mm comprising 100 cavities loaded with filled 1-3 mL medical containers, as determined by finite element analysis (FEA). By contrast, a conventional holding device including 100 cavities, but without surfaces of different elevations, was found by FEA to deflect by approximately4.3mm.

As previously described, the holding devices 10 of the present disclosure are configured to be disposed in a container, such as a tub. Exemplary tubs are described in the '358 patent, the '793 patent, and the '715 publication referred to previously. For example, the holding devices 10 of the present disclosure can be disposed within the tub, such as on a ledge formed on an interior surface of the tub. The medical containers can be inserted through through-holes or openings of the holding devices 10, such that distal portions of the medical containers (i.e., portions of the medical containers that are farthest away from the portion of the device or container grasped or manipulated by a user) are in the interior of the tub and a sidewall of the medical container is supported by the through-hold or opening of the holding devices 10.

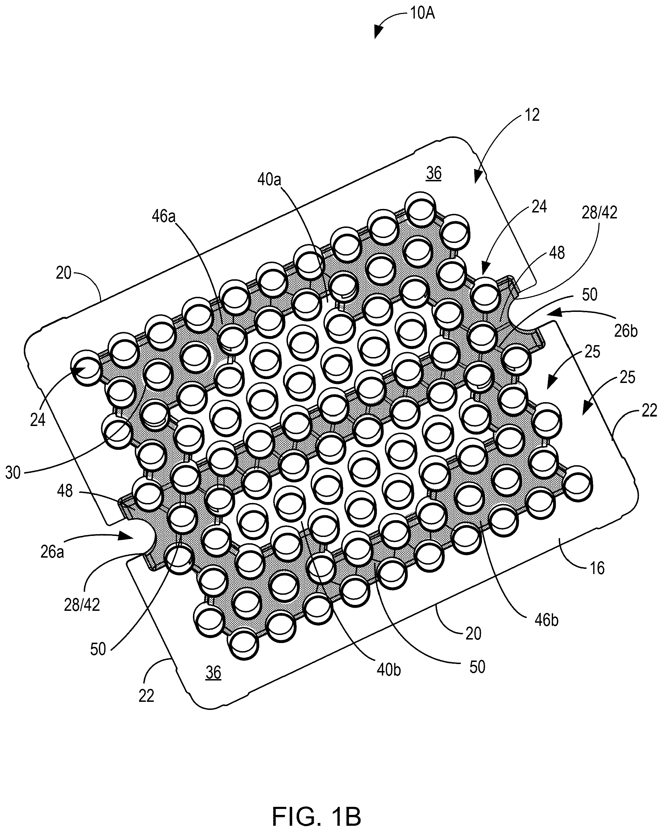

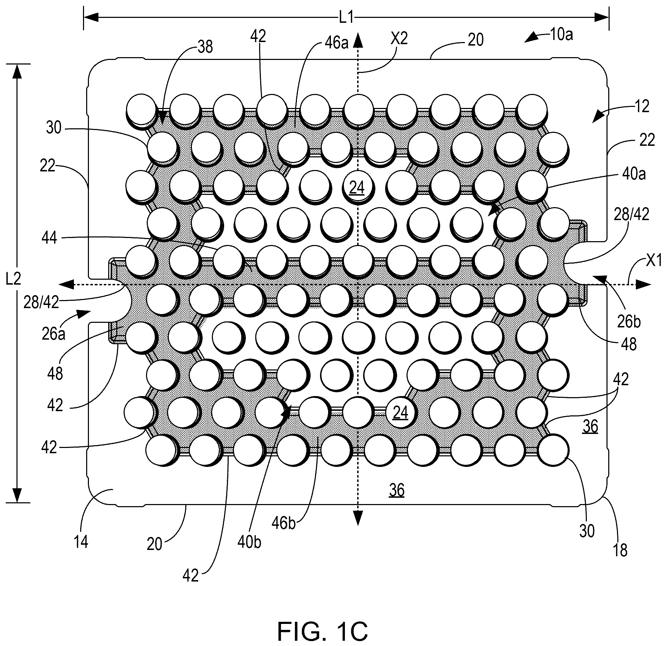

FIGS. 1A-1E illustrate an exemplary holding device 10a configured to support medical containers including features of the present disclosure. The holding device 10a comprises a supporting plate 12 having a first or upper surface 14, a second or lower surface 16, and a peripheral edge 18 extending about the supporting plate 12 between the upper surface 14 and the lower surface 16. In some examples, the supporting plate 12 is substantially rectangular in shape, with the peripheral edge 18 thus including a first pair of opposed sides 20 and a second pair of opposed sides 22. In some embodiments, the first pair of opposed sides 20 may be defined as longitudinal sides (hereafter, "longitudinal sides 20") and the second pair of opposed sides 22 may be defined as transverse sides (hereafter, "transverse sides 22"). As used herein, the longitudinal sides refer to longer sides 20 of the supporting plate 12 having a length L1 (shown in FIG. 1C) and the transverse sides refer to shorter sides 22 of the supporting plate 12 having a length L2, which is shorter than the length L1. The supporting plate 12 may also be square, in which case the longitudinal sides 20 and the transverse sides 22 are the same length (i.e., L1 is equal to L2). As shown in FIG. 1B, the substantially rectangular supporting plate 12 defines a first axis X1 (also referred to as a longitudinal, horizontal, or x axis) extending parallel to the longitudinal sides 20 of the supporting plate 12 and a second axis X2 (also referred to as a transverse, latitudinal, vertical, or y axis) extending parallel to the transverse sides 22 of the supporting plate 12.

Dimensions of the supporting plate 12 will be determined by those skilled in the art based on the number of medical containers that can be supported by the holding device 10a, dimensions of the medical containers, and/or dimensions of the tub or container to which the holding device 10a is mounted. In some examples, dimensions of the supporting plate 12 can be determined based on published standards for pre-filled syringe packaging dimensions (e.g., ISO 11040-7:2015(E): Packaging systems for sterilized sub-assembled syringes ready for filling).

The supporting plate 12 further comprises multiple openings 24 formed through the supporting plate 12 configured to receive the medical containers. The openings 24 are sized to support to medical containers in an upright position. For example, the openings 24 can be wide enough so that a sidewall of the medical container passes through the openings 24, while preventing a flange or another protruding portion of the medical container from passing through the openings 24. The dimensions of the openings 24 are generally dependent upon a size of the medical container intended to be used with the holding device 10a. For example, for a supporting plate 12 comprising one-hundred openings 24, the inner diameter of the openings 24 can be from about 12.1 mm to about 12.35 mm.

As shown in FIGS. 1A and 1B, the openings 24 can be arranged on the supporting plate 12 as a plurality of rows 25 that extend parallel to the longitudinal or first axis X1 of the supporting plate 12. For example, the supporting plate 12 can include ten rows, with each of the rows 25 including ten openings 24. The openings 24 of each row 25 can be equidistantly spaced from each other, with the spacing dependent on the number of openings 24 included in each row 25. Each row 25 of openings 24 can be offset from its adjacent rows 25 along the longitudinal or first axis X1 in an alternating pattern, such that the openings 24 of adjacent rows 25 are staggered relative to each other. This may result in what is termed as a "hexagonal arrangement" of openings 24 in supporting plate 12, as each opening 24 (other than the outer-most "perimeter" openings of holding device 10a) will have an arrangement of six adjacent openings 24 arranged hexagonally thereabout.

The supporting plate 12 can also include cutouts 26a, 26b extending inwardly from other portions of the peripheral edge 18. For example, the supporting plate 12 may include a first cutout 26a on one of the transverse sides 22 of the supporting plate 12 and a second cutout 26b on a second transverse side 22 of the supporting plate 12. In some examples, the cutouts 26a, 26b are a u-shaped gap or opening extending inwardly from the peripheral edge 18 of the supporting plate 12. The holding device 10a can also include a curved wall 28 extending from the upper surface 14 and/or the lower surface 16 of the supporting plate 12 and about at least a portion of each of the first cutout 26a and the second cutout 26b, with the curved wall(s) 28 providing finger grips by which a user can grasp the holding device 10a. The first and second cutouts 26a, 26b may be offset from each other along the transverse or second axis X2 of the supporting plate 12, as may be dictated by the offsetting of alternating rows of openings 24 along the longitudinal or first axis X1.

The holding device 10a further comprises tubular walls, members, or ridges (referred to herein as chimneys 30) protruding from the upper surface 14 and/or the lower surface 16 of the supporting plate 12 - with the chimneys shown in FIGS. 1A-1E as protruding from upper surface 14. Each chimney 30 encloses or partially encloses one of the openings 24 and is positioned to guide insertion of the medical containers into the opening 24. The chimneys 30 can include a tubular wall comprising a cylindrical inner surface 32 having a diameter substantially the same as the diameter of the opening 24 enclosed or partially enclosed by the chimney 30 and a cylindrical outer surface 34 opposite the inner surface 32 having an outer diameter.

As previously described, the holding device 10a includes structures for increasing stiffness of the supporting plate 12 and for resisting deflection or deformation when the holding device 10a is fully filled with medical containers. In particular, the holding device 10a and supporting plate 12 are configured to ensure that downward deflection of the holding device 10a, when filled with medical containers, is 2.5 mm or less. In order to increase stiffness of the supporting plate 12, the supporting plate 12 can include portions of different elevations connected by angled or vertical walls. For example, as shown in FIGS. 1A-1E, the supporting plate 12 can include a peripheral lower portion 36 extending inward from the peripheral edge 18 of the supporting plate 12. The supporting plate 12 also include one or more elevated or raised portions or plateaus 38 enclosed by the peripheral lower portion 36, along with inner lower portions 40 enclosed by the raised plateau(s) 38, with upwardly angled or vertical connecting walls 42 (hereafter "connecting walls 42") extending between the peripheral and inner lower portions 36, 40 and the raised plateau(s) 38. The openings 24 and/or chimneys 30 extend through or from the lower portions 36, 40, raised plateau(s) 38, and/or connecting walls 42 of the supporting plate 12. For example, outer openings 24 and outer chimneys 30 (i.e., openings 24 and chimneys 30 proximate to the peripheral edge 18 of the supporting plate 12) can pass through or extend from the peripheral lower portion 36, the raised plateau(s) 38, and the connecting walls 42 of the supporting plate 12. Inner openings 24 and inner chimneys 30 (i.e., openings 24 and chimneys 30 proximate to a center of the supporting plate 12 and enclosed by the outer openings 24 and/or outer chimneys 30) can pass through or extend from the raised plateau 38, the inner lower portions 40, and the connecting walls 42 of the supporting plate 12.

The peripheral lower portion 36 and inner lower portions 40 of the supporting plate 12 refer to a lowest portion of the holding device 10a positioned such that, if the holding device 10a is placed on a flat substrate, the peripheral lower portion 36 and inner lower portions 40 of the supporting plate 12 (on the lower or second surface 16) are in face-to-face contact with the flat substrate. The connecting walls 42 and the raised plateau 38 of the supporting plate 12 are elevated relative to the peripheral lower portion 36 and inner lower portions 40, meaning that, when positioned on the flat substrate, the connecting walls 42 of the supporting plate 12 and the raised plateau 38 of the supporting plate 12 are not in face-to-face contact with the flat substrate.

In some examples, the peripheral lower portion 36 is flat, meaning that the peripheral lower portion 36 of the supporting plate 12 can rest against or be mounted to portions of the tub or container for securing the holding device 10a to the tub or container. In some examples, the holding device 10a can be configured to rest against and/or be supported by parallel rails (e.g., rails extending parallel to either the longitudinal or first axis X1 or the transverse or second axis X2 of the supporting plate 12). In that case, the parallel rails can be placed under the flat surface of the peripheral lower portion 36 proximate to either the longitudinal sides 20 or the transverse sides 22 of the holding device 10a. Desirably, the supporting plate 12 is sufficiently stiff to maintain its shape and to resist deformation or deflection even when only supported by the two parallel rails.

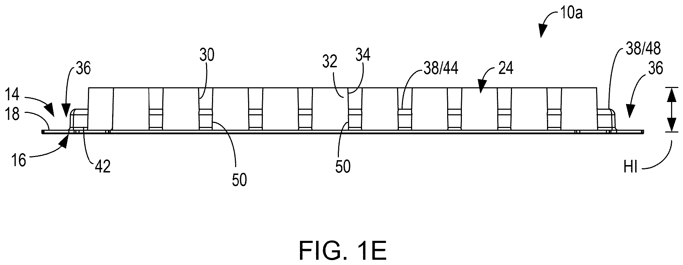

In some examples, the raised plateau(s) 38 of the supporting plate 12 can be elevated relative to the peripheral lower portion 36 and lower inner portions 40 by a height H1 (shown in FIG. 1A and 1E), which can be from about 2.0 mm to about a top of the chimneys 30. In some embodiments, and as shown in FIGS. 1A-1E, the height H1 of the raised plateau(s) 38 can be less than the height of the chimneys 30 - with the height of the chimneys 30 defined as a height from the first or upper surface 14 of the peripheral lower portion 36 or inner lower portions 40 to a top of the chimney 30. For example, the height H1 of the raised plateau(s) 38 can be between 10% and 90% of the full height of the chimneys 30. In other examples, the height H1 of the raised plateau(s) 38 may be about the same as a height of the chimneys 30.

The connecting walls 42 of the supporting plate 12 extend between the peripheral lower portion 36 and the raised plateau(s) 38 and between the inner lower portions 40 and the raised plateau(s) 38. As previously described, the connecting walls 42 can be connected to and/or extend from some of the some chimneys 30 of the holding device 10a. For example, the connecting walls 42 can intersect and/or extend from at least two adjacent chimneys 30 of the holding device 10a. In some examples, as shown in FIGS. 1A-1E, the connecting walls 42 comprise substantially vertical walls extending substantially orthogonally relative to the first or upper surface 14 of the peripheral lower portion 36 and/or inner lower portions 40. In some examples, the connecting walls 42 can have a slight slope, often referred to as a draft angle, to facilitate removing the holding device 10a from a tool or mold following injection molding. For example, the slight slope or draft angle can be between 0.1 degree and 4.0 degrees, and preferably about 1.5 degrees or less.

According to aspects of the disclosure, the supporting plate 12 can include different arrangements or configurations of lower portions 36, 40, raised plateau(s) 38, and connecting walls 42. The different arrangements or configurations can be selected by those skilled in the art to impart increased stiffness to different portions or sides of the supporting plate 12 and/or to provide additional support (i.e., resistance to deflection) for portions of the supporting plate 12 that are expected to be exposed to substantial forces during use.

In the embodiment of FIGS. 1A-1E, the raised plateau(s) 38 is/are formed as a single, continuous raised plateau, but for purposes of description may be understood to include different portions or regions that may generally be characterized as a center plateau 44, peripheral plateaus 46, and two C-shaped plateaus 48. The arrangement of the center plateau 44, peripheral plateaus 46, and C-shaped plateaus 48 imparts an increased stiffness (and increased resistance to deflection) to a center region of the supporting plate 12, as well about a periphery of supporting plate 12.

The center plateau 44 is configured to have a generally linear configuration and extends between outermost chimneys 30 on opposing transverse sides 22 of the supporting plate 12. The center plateau 44 is formed between (and to encompass) the two centermost rows 25 of openings 24/chimneys 30, so as to be aligned along the longitudinal axis X1. Connecting walls 42 of the supporting plate 12 extend up from inner lower portions 40 and up to the raised plateaus 38, with the connecting walls 42 in parallel with the longitudinal axis X1.

The peripheral plateaus 46 are formed on opposing sides of the center plateau 44 - with a first peripheral plateau 46a thus formed on one side of the longitudinal axis X1 and a second peripheral plateau 46b formed on the other side of the longitudinal axis X1. Each of the peripheral plateaus 46 is formed to generally follow a path of the peripheral or outer chimneys 30 of the holding device 10a. The peripheral plateaus 46 - along with center plateau 44 - thus generally encircle inner lower portions 40 of the supporting plate 12, with the first peripheral plateau 46a and the center plateau 44 encircling a first inner lower portion 40a and the second peripheral plateau 46b and the center plateau 44 encircling a second inner lower portion 40b. The first peripheral plateau 46a and the second peripheral plateau 46b may be mirror images of each other on opposing sides of the center plateau 44 (i.e., on opposing sides of longitudinal axis X1), with the first inner lower portion 40a and the second inner lower portion 40b thus also being mirror images of each other. The increased stiffness provided to opposing sides of the supporting plate 12 by the first peripheral plateau 46a and the second peripheral plateau 46b may thus be identical.

Each of the peripheral plateaus 46 may have a generally linear/straight profile on a side thereof that is proximate to a respective longitudinal side 20 of the supporting plate 12 (running parallel to the longitudinal axis X1), with this side of the peripheral plateau 46 aligned with an outermost row of openings 24/chimneys 30. A corresponding connecting wall 42 extends between the peripheral plateau 46 and the peripheral lower portion 36 along this portion/side of the peripheral plateau 46, with the connecting wall 42 intersected by the outermost row of openings 24/chimneys 30.

Each of the peripheral plateaus 46 may then have a zig-zag profile on opposing sides thereof that are proximate to respective transverse sides 22 of the supporting plate 12. The zig-zag profile of the peripheral plateaus 46 on these sides follows the arrangement/positioning of the outermost openings 24/chimneys 30 on the transverse sides 22 of the supporting plate 12. A plurality of connecting walls 42 extend between the peripheral plateau 38 and the peripheral lower portion 36 along these sides of the peripheral plateaus 46, with each connecting wall 42 formed between adjacent outer openings 24/chimneys 30 - such that the connecting walls 42 also have a zig-zag pattern following that of the zig-zag profile of the peripheral plateaus 46. That is, the connecting walls 42 include alternating inwardly directed walls 42 and outwardly directed walls 42.

The two C-shaped plateaus 48 of supporting plate 12 are provided at opposing ends of the center plateau 44, with the C-shaped plateaus 48 extending out from the center plateau 44 and out past the peripheral plateaus 46 into/toward the peripheral lower portion 36 of supporting plate 12. The C-shaped plateaus 48 are positioned so as to be aligned with the first cutout 26a and the second cutout 26b on opposing transverse sides 22 of the supporting plate 12, with the C-shaped plateaus 48 thus offset from each other relative to the longitudinal or first axis X1 of the supporting plate 12 (i.e., offset in the direction of the transverse or second axis X2), as dictated by the offsetting of alternating rows of openings 24/chimneys 30 along the longitudinal or first axis X1. The C-shaped plateaus 48 extend outwardly past the outer openings 24/chimneys 30 and to the first and second cutouts 26a, 26b, such that the curved walls 28 extending from the upper surface 14 of the supporting plate 12 and about at least a portion of the respective cutouts 26a, 26b form connecting walls 42 that extend up from the peripheral lower portion 36 (around cutouts 26a, 26b) to the C-shaped plateaus 48. Additional connecting walls 42 also extend up from the peripheral lower portion 36 to the C-shaped plateaus 48 on each of opposing sides of the curved walls 28, to further define the C-shaped plateaus 48.

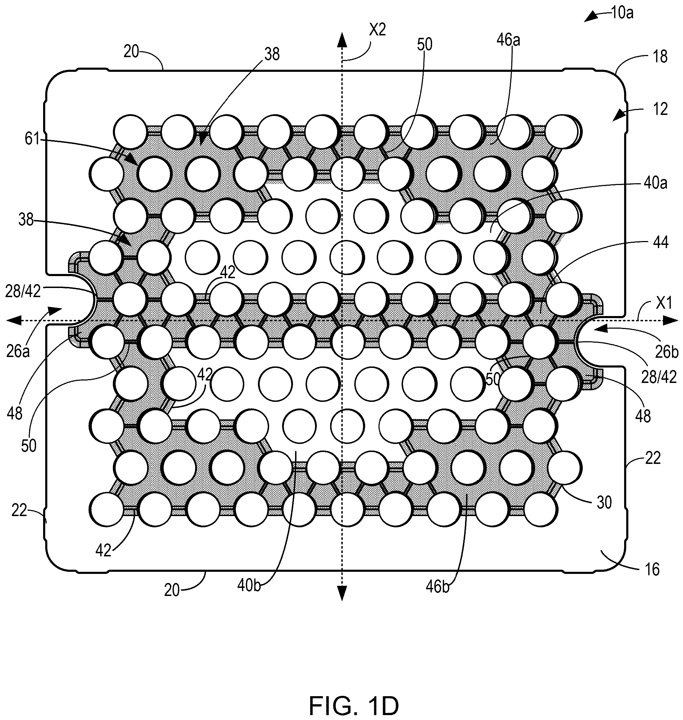

According to some aspects of the disclosure, and as best shown in FIGS. 1B and 1D, the holding device 10a may further include a plurality of radial reinforcing ribs 50 that connect adjacent chimneys 30 one to each other for providing still additional rigidity to the holding device 10a. The ribs 50 may extend between at least some of the chimneys 30 that extend up through the raised plateau(s) 38 of the holding device 10a, to provide additional rigidity to the holding device 10a at the areas of the raised plateau(s) 38. According to embodiments, each of the ribs 50 is structured as a planar, wall-shaped member that is oriented vertically and orthogonal to the supporting plate 12. Each rib 50 extends between an adjacent pair of chimneys 34, with the rib 50 being formed integrally with the chimneys 34 as part of a single structure. The ribs 50 are constructed to extend up from a bottom end of chimneys 34 to a bottom surface 16 of the raised plateau(s) 38.

According to one embodiment, ribs 50 are provided between all of the chimneys 30 that extend up through the raised center plateau 44 and between all of the chimneys 30 that extend up through the C-shaped plateaus 48. The ribs 50 provide additional rigidity to the holding device 10a at the areas of the center plateau 44 and the C-shaped plateaus 48, as it is recognized that these areas of the supporting plate 12 may experience a higher load when the holding device 10a is loaded with medical containers. Additionally, ribs 50 may also be provided between at least some of the chimneys 30 that extend up through the peripheral plateaus 46, such as for at least some of the outer chimneys 30 that are proximate the longitudinal sides 20 of supporting plate 12.

With the holding device 10a structured to include the raised plateau(s) 38 as shown in FIGS. 1A-1E, the deflection of the supporting plate may be reduced to less than 2.5 mm in a vertical direction. In particular, the deflection of the supporting plate in the vertical direction at the center of the plate may be 1.40 mm.

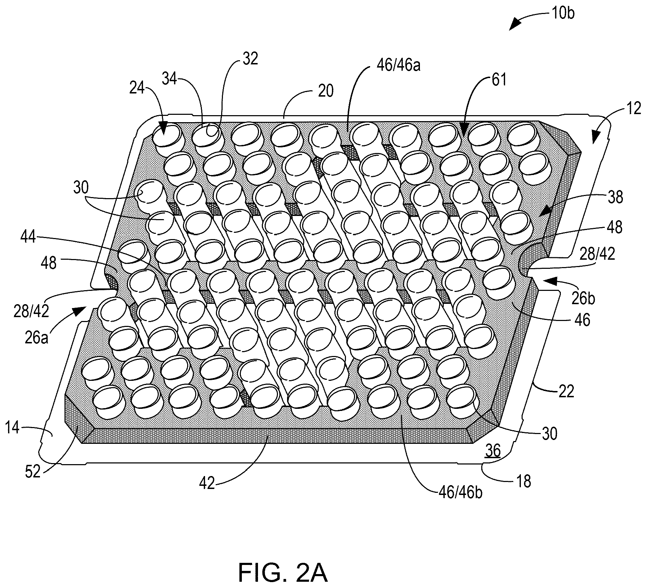

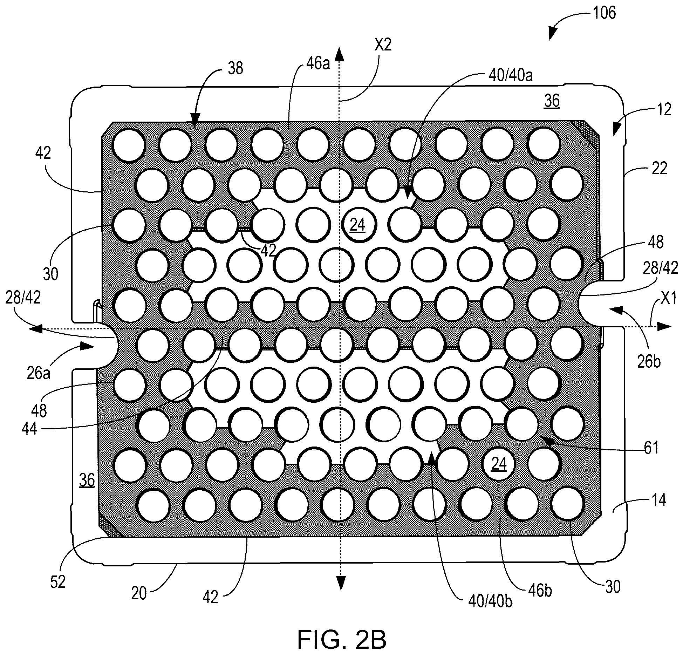

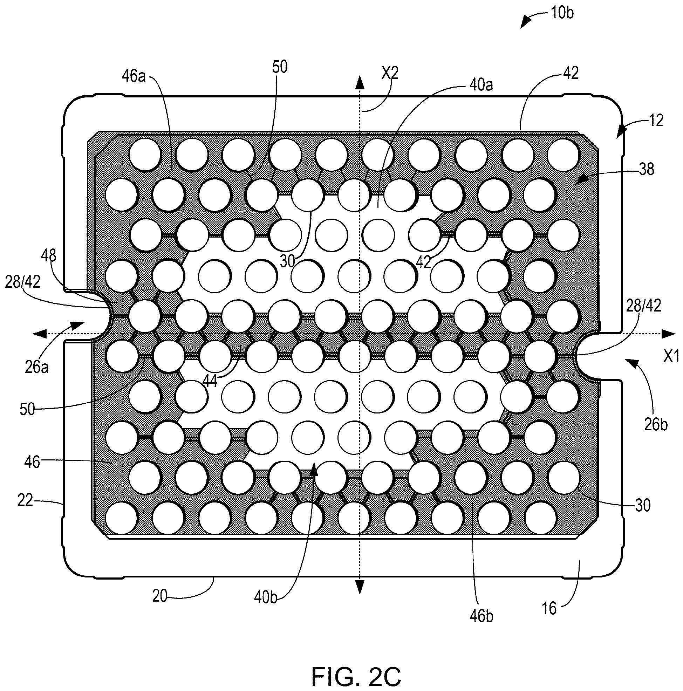

FIGS. 2A-2C illustrate another example of a holding device 10b that includes structures for increasing stiffness of the supporting plate 12 and for resisting deflection or deformation when the holding device 10b is fully filled with medical containers. Similar to the holding device 10a of FIGS. 1A-1E, the holding device 10b includes a supporting plate 12 having a peripheral lower portion 36, raised plateau(s) 38 enclosed by the peripheral lower portion 36, inner lower portions 40 enclosed by the raised plateau(s) 38, and upwardly angled or connecting walls 42 ("connecting walls 42") extending between the peripheral and inner lower portions 36, 40 and the raised plateau(s) 38. The raised plateau(s) 38 may again be formed as a single, continuous raised plateau, but for purposes of description may be understood to include different portions or regions that may generally be characterized as a center plateau 44, peripheral plateaus 46, and two C-shaped plateaus 48. However, the structure of the peripheral plateaus 46 in holding device 10b differs from those included in the holding device 10a.

As shown in FIGS. 2A-2C, the peripheral plateaus 46 in holding device 10b are formed on opposing sides of the center plateau 44 - with a first peripheral plateau 46a thus formed on one side of the longitudinal axis X1 and a second peripheral plateau 46b formed on the other side of the longitudinal axis X1. The peripheral plateaus 46 - along with center plateau 44 - thus generally encircle inner lower portions 40 of the supporting plate 12, with the first peripheral plateau 46a and the center plateau 44 encircling a first inner lower portion 40a and the second peripheral plateau 46b and the center plateau 44 encircling a second inner lower portion 40b. The first peripheral plateau 46a and the second peripheral plateau 46b may be mirror images of each other on opposing sides of the center plateau 44 (i.e., on opposing sides of longitudinal axis X1), with the first inner lower portion 40a and the second inner lower portion 40b thus also being mirror images of each other. The increased stiffness provided to opposing sides of the supporting plate 12 by the first peripheral plateau 46a and the second peripheral plateau 46b may thus be identical.

As shown in FIGS. 2A- 2C, each of the peripheral plateaus 46 is formed to extend out past the peripheral or outermost chimneys 30 of the holding device 10b (i.e., outside a receiving region 61 for receiving medical containers defined by the chimneys 30) and farther out toward the peripheral lower portion 36 - such that a width of the peripheral lower portion 36 on supporting plate 12 is reduced in the holding device 10b as compared to the holding device 10a. With the peripheral plateaus 46 extending out past the peripheral or outer chimneys 30, the peripheral plateaus 46 may be considered as incorporating therein the C-shaped plateaus 48 that are formed about first and second cutouts 26a, 26b of the supporting plate 12.

With the peripheral plateaus 46 extending out past the peripheral or outer chimneys 30, each of the peripheral plateaus 46 may have a generally linear/straight profile on each side thereof - i.e., on the side that is proximate to a respective longitudinal side 20 of the supporting plate 12 (running parallel to the longitudinal axis X1) and on the sides that are proximate to the transverse sides 22 of the supporting plate 12 (running parallel to the transverse axis X2). On each of these sides of the peripheral plateaus 46, a corresponding connecting wall 42 is provided that extends between the peripheral plateau 38 and the peripheral lower portion 36. A connecting wall 42 having a linear/planar construction may thus be provided for each peripheral plateau 38 on the side that is proximate to a respective longitudinal side 20 of the supporting plate 12, and connecting walls 42 having a linear/planar construction may also be provided for each peripheral plateau 38 on the sides that are proximate to the transverse sides 22 of the supporting plate 12. In some embodiments, a chamfered corner 52 may be provided where the connecting walls 42 intersect, with a connecting wall 42 provided that is angled (relative to the X1 and X2 axes). Also, in some embodiments, one or more of the chamfered corners 52 formed by the connecting wall 42 may be configured as a sloped wall segment that is angled, for example, at 60 degrees from the supporting plate 12, to facilitate removing the holding device 10b from a tool or mold following injection molding.

In some embodiments, and as previously described for holding device 10a, the holding device 10b may further include a plurality of radial reinforcing ribs 50 that connect adjacent chimneys 30 one to each other for providing still additional rigidity to the holding device 10b. The ribs 50 may extend between at least some of the chimneys 30 that extend up through the raised plateau(s) 38 of the holding device 10, to provide additional rigidity to the holding device 10b at the areas of the raised plateau(s) 38. In particular, ribs 50 are provided between all of the chimneys 30 that extend up through the raised center plateau 44 and may also be provided between at least some of the chimneys 30 that extend up through the peripheral plateaus 46 and through the C-shaped plateaus 48.

With the holding device 10b structured to include raised plateau(s) 38 as shown in FIGS. 2A-2C, where peripheral plateaus 46 extend out past the region of openings 24/chimneys 30, the deflection of the supporting plate may be reduced to less than 2.5 mm in a vertical direction. In particular, the deflection of the supporting plate in the vertical direction at the center of the plate may be 0.9 mm.

FIGS. 3A-3C illustrate another example of a holding device 10c that includes structures for increasing stiffness of the supporting plate 12 and for resisting deflection or deformation when the holding device 10c is fully filled with medical containers. More specifically, the holding device 10c includes a supporting plate 12 having a peripheral lower portion 36, a raised cross-shaped plateau 38 enclosed by the peripheral lower portion 36, inner lower portions 40, upwardly angled or connecting walls 42 ("connecting walls 42") extending between the peripheral and inner lower portions 36, 40 and the raised cross-shaped plateau 38, and a peripheral wall 54 formed separately from the cross-shaped plateau 38.

The raised cross-shaped plateau 38 may be characterized as including a first plateau arm 56 and a second plateau arm 58. The raised cross-shaped plateau 38 is formed so as to be centered on supporting plate 12 - with the first plateau arm 56 thus aligned along the longitudinal axis X1 and the second plateau arm 58 thus aligned along the transverse axis X2. Each of the first plateau arm 56 and the second plateau arm 58 may have a width that encompasses at least several openings 24/chimneys 30 of the holding device 10c. For example, each of the first plateau arm 56 and the second plateau arm 58 may have a width that encompasses portions of three or four chimneys. The first plateau arm 56 and the second plateau arm 58 - along with peripheral wall 54 - define four (4) inner lower portions 40 of the supporting plate 12, with these inner lower portions 40 located in four quadrants of the supporting plate 12.

At each of opposing ends of the first plateau arm 56, the raised cross-shaped plateau 38 may have a C-shaped configuration or portion 60 (i.e., a C-shaped plateau), similar to as previously described with the C-shaped plateaus 48 of holding device 10a. The C-shaped portions 60 are positioned so as to be aligned with the first cutout 26a and the second cutout 26b on opposing transverse sides 22 of the supporting plate 12, with the C-shaped portions 60 thus offset from each other relative to the longitudinal or first axis X1 of the supporting plate 12 (in the direction of the transverse or second axis X2), as dictated by the offsetting of alternating rows of openings 24/chimneys 30 along the longitudinal or first axis X1. The C-shaped portions 60 extend outwardly past the outer openings 24/chimneys 30 and to the first and second cutouts 26a, 26b, such that the curved walls 28 extending from the upper surface 14 of the supporting plate 12 and about at least a portion of the respective cutouts 26a, 26b form connecting walls 42 that extend up from the peripheral lower portion 36 to the C-shaped plateaus 48.

As previously described, the connecting walls 42 of the supporting plate 12 can be connected to and/or extend from some of the some chimneys 30 of the holding device 10c. For example, the connecting walls 42 can intersect and/or extend from at least two adjacent chimneys 30 of the holding device 10c. A portion of the connecting walls 42 extend between the peripheral lower portion 36 and the raised cross-shaped plateau 38. As shown in FIGS. 3A-3C, the connecting walls 42 at each of the opposing ends of the second plateau arm 58 intersect and/or extend from the chimneys 30 at these locations, while the connecting walls 42 at each of the opposing ends of the first plateau arm 56 are positioned out past the chimneys 30 (such that they are not intersected by chimneys 30). Another portion of the connecting walls 42 extend between the inner lower portions 40 and the raised cross-shaped plateau 38, such as at locations along a length of the first plateau arm 56 and the second plateau arm 58.

The supporting plate 12 also includes a peripheral wall 54 that is distinct from the connecting walls 42 in that it does not connect the raised plateau 38 to the lower portions 36, 40 of supporting plate 12. The peripheral wall 54 generally separates the peripheral lower portion 36 from a region of supporting plate 12 that includes the openings 24/chimneys 30 and also surrounds the raised cross-shaped plateau 38. In the illustrated embodiment, the peripheral wall 54 is aligned with the outer chimneys 30 of the holding device 10c - with the peripheral wall 54 intersected by the outermost row of openings 24/chimneys 30. The peripheral wall 54 is joined to the raised cross-shaped plateau 38 at each of the first plateau arm 56 and the second plateau arm 58 - with the peripheral wall 54 joined to the ends of the second plateau arm 58 and with the peripheral wall 54 intersecting the first plateau arm 56 at locations thereof that are aligned with the outer chimneys 30 on the transverse sides 22 of the supporting plate 12. At the ends of the second plateau arm 58, the peripheral wall 54 is integrally joined to the connecting walls 42 provided thereat that extend between the raised cross-shaped plateau 38 and the peripheral lower portion 36 (an intersect or extend from chimneys 30).

The peripheral wall 54 may be understood to function similarly to the ribs 50 previously described for holding devices 10a, 10b in that the peripheral wall 54 connects adjacent outer chimneys 30 one to each other for providing additional rigidity to the holding device 10c. The peripheral wall 54 is thus structured as a planar, wall-shaped member that is oriented vertically and orthogonal to the supporting plate 12. The peripheral wall 54 extends between adjacent pairs of outer chimneys 34, with the peripheral wall 54 being formed integrally with the chimneys 34 as part of a single structure. The peripheral wall 54 is constructed to extend up from a bottom end of chimneys 34 to a height that is equal to a top surface of the raised cross-shaped plateau 38 (which may be less than a height of the chimneys 30, in some embodiments).

With the holding device 10c structured to include the raised cross-shaped plateau 38 and the peripheral wall 54 as shown in FIGS. 3A-3C, the deflection of the supporting plate may be reduced to equal to or less than 2.5 mm in a vertical direction. In particular, the deflection of the supporting plate in the vertical direction at the center of the plate being 2.5 mm.

FIGS. 4A-4C illustrate another example of a holding device 10d that includes structures for increasing stiffness of the supporting plate 12 and for resisting deflection or deformation when the holding device 10d is fully filled with medical containers. Similar to the holding device 10c of FIGS. 3A-3C, the holding device 10d includes a supporting plate 12 having a peripheral lower portion 36, a raised cross-shaped plateau 38 enclosed by the peripheral lower portion 36, inner lower portions 40, upwardly angled or connecting walls 42 ("connecting walls 42") extending between the peripheral and inner lower portions 36, 40 and the raised cross-shaped plateau 38, and a peripheral wall 54 formed separately from the cross-shaped plateau 38. However, the structure and positioning of the raised cross-shaped plateau 38 and the peripheral wall 54 in holding device 10d differs from those included in the holding device 10c.

As shown in FIGS. 4A-4C, the cross-shaped plateau 38 of supporting plate 12 is formed such that each of the first plateau arm 56 and the second plateau arm 58 extend out past the peripheral or outermost chimneys 30 of the holding device 10d. Similar to the cross-shaped plateau 38 of holding device 10c, the opposing ends of the first plateau arm 56 may have C-shaped portions 60 positioned so as to be aligned with the first cutout 26a and the second cutout 26b, respectively, on opposing transverse sides 22 of the supporting plate 12, with the C-shaped portions 60 extending outwardly past the outer openings 24/chimneys 30 and to the first and second cutouts 26a, 26b. However, in holding device 10d, the second plateau arm 58 of raised cross-shaped plateau 38 is configured such that the opposing ends thereof also extend outwardly past the outer openings 24/chimneys 30.

With the cross-shaped plateau 38 configured such that each of the first plateau arm 56 and the second plateau arm 58 extend out past the outer chimneys 30 of the holding device 10d, the location of the peripheral wall 54 on supporting plate 12 is shifted outward, so as to align with the ends of the first plateau arm 56 and the second plateau arm 58. The peripheral wall 54 is thus positioned out past the peripheral or outermost chimneys 30 of the holding device 10d (i.e., outside of receiving region 61), such that each of the sidewalls of the peripheral wall 54 may have a generally linear/straight profile that is not intersected by any of the chimneys 30. On sides of the supporting plate 12 proximate the longitudinal sides 20, the peripheral wall 54 is a straight wall along the entire length thereof, while on sides of the supporting plate 12 proximate the transverse sides 22, the peripheral wall 54 is straight except for a location where it intersects with the C-shaped portions 60 of first plateau arm 56. The peripheral wall 54 is joined to the raised cross-shaped plateau 38 at the ends of each of the first plateau arm 56 and the second plateau arm 58. At the ends of the first plateau arm 56 and the second plateau arm 58, the peripheral wall 54 is integrally joined to the connecting walls 42 provided thereat that extend between the raised cross-shaped plateau 38 and the peripheral lower portion 36.

In some embodiments, the cross-shaped plateau 38 may be further configured such that each of the first plateau arm 56 and the second plateau arm 58 includes two bump-out plateaus 62 formed thereon that are further raised/elevated as compared to the remainder of the first plateau arm 56 and the second plateau arm 58. The bump-out plateaus 62 may be spaced apart from one another and located towards opposing ends of the first plateau arm 56 and the second plateau arm 58. Each of the bump-out plateaus 62 may be further elevated such that a top surface thereof is at a same height as the height of the chimneys 30. Each of the bump-out plateaus 62 may be formed between a grouping of three (3) adjacent chimneys 30, so as to have a generally triangular shape. The bump-out plateaus 62 may function to add still additional rigidity to the supporting plate 12.

With the holding device 10d structured to include the raised cross-shaped plateau 38 and the peripheral wall 54 as shown in FIGS. 4A-4C, the deflection of the supporting plate 12 may be reduced to less than 2.5 mm in a vertical direction. In particular, the deflection of the supporting plate in the vertical direction at the center of the plate being 2.1 mm.

According to aspects of the disclosure, the holding devices 10a-10d of the present disclosure can be made of various plastic materials commonly used for disposable medical packaging, containers, and/or devices. Further, the holding devices 10a-10d can be made by various forming and molding processes, as are known in the art, with the holding devices 10a-10d formed as a single, integral component via such processes. For example, the holding devices 10a-10d of the present disclosure can comprise a thermoplastic material, preferably polypropylene, or polyester, polycarbonate, polyethylene, polyethylene terephthalate, acrylonitrile butadiene styrene, or other injection moldable or formable resin materials, as are known in the art. In some examples, the supporting plate 12 and chimneys 30 can be integrally formed from a same material, such as from the same thermoplastic material. In some examples, the holding devices 10a-10d are formed by a single injection molding process, in which the holding device 10a-10d is formed by injecting a fluid polymer material between a first tool and a second tool in a single process. In order to optimize the holding devices 10a-10d for injection molding, in some examples, vertical or substantially vertical surfaces of the holding device 10a-10d, such as the previously described walls 42, comprising connecting walls 42, as well as the inner surface 32 and/or the outer surface 34 of the chimneys 30, can include a slightly sloped surface having a draft angle (e.g., an angle of about 0.1 degree to 4.0 degrees, for example, 1.5 degree). Including substantially vertical surfaces with a slight slope or draft angle makes the molded part easier to remove from the mold following injection molding. In other examples, the holding devices 10a-10d can be made by three-dimensional printing by processes known in the art.

Beneficially, embodiments of the disclosure thus provide holding devices that exhibit increased stiffness in order to reduce deflection thereof during transport and filling. Avoiding deformation can improve accuracy of the filling process by avoiding misalignment between the medical containers contained by the tray or nest and the automated filling machines. The holding devices of the present disclosure are intended to provide such increased stiffness and to resist deflection or deformation under weight of multiple medical containers contained thereby, with one or more raised plateaus being formed on the supporting plate of the holding device to increase the rigidity thereof.

While examples of the holding devices are shown in the accompanying figures and described hereinabove in detail, other examples will be apparent to, and readily made by, those skilled in the art without departing from the scope and spirit of the invention. Accordingly, the foregoing description is intended to be illustrative rather than restrictive. The invention described hereinabove is defined by the appended claims and all changes to the invention that fall within the meaning and the range of equivalency of the claims are to be embraced within their scope.

## Claims

1. A holding device configured to support medical containers, the holding device comprising:
a supporting plate comprising a first surface, a second surface, a peripheral edge between the first surface and the second surface and including a first pair of opposed sides and a second pair of opposed sides, and a plurality of openings through the supporting plate configured to receive the medical containers, wherein the supporting plate comprises:
a peripheral lower portion extending inward from the peripheral edge;
a pair of cutouts formed in the second pair of opposed sides and in the peripheral lower portion;
one or more raised plateaus vertically offset from the peripheral lower portion and enclosed by the peripheral lower portion; and
connecting walls extending between the peripheral lower portion and the one or more raised plateaus;
wherein the one or more raised plateaus include a pair of C-shaped plateaus positioned, respectively, about the pair of cutouts; and
a plurality of chimneys protruding from the first surface of the supporting plate and at least partially enclosing the plurality of openings, such that the plurality of chimneys guide insertion of the medical containers into the plurality of openings.

2. The holding device of claim 1, wherein the plurality of openings are arranged on the supporting plate as a plurality of rows aligned parallel to a longitudinal axis of the supporting plate, and wherein each adjacent pair of rows of the plurality of row are offset from each other relative to the longitudinal axis, such that the plurality of rows are staggered.

3. The holding device of claim 2, wherein the one or more raised plateaus comprises a continuously formed plateau comprising a plurality of connected plateau regions, the plurality of connected plateau regions comprising:
a center plateau having a linear configuration and being formed between two centermost rows of the plurality of rows, so as to be aligned along the longitudinal axis;
a first peripheral plateau and a second peripheral plateau positioned on opposing sides of the center plateau; and
the pair of C-shaped plateaus positioned on opposing ends of the center plateau.

4. The holding device of claim 3, wherein the center plateau and the first peripheral plateau encircle a first inner lower portion of the supporting plate and the center plateau and the second peripheral plateau encircle a second inner lower portion of the supporting plate, the first inner lower portion and the second inner lower portion vertically aligned with the peripheral lower portion, and wherein the connecting walls extend between the first and second inner lower portions and the center plateau and the first and second peripheral plateaus.

5. The holding device of claim 3 or claim 4, wherein the connecting walls extending between the peripheral lower portion and the first and second peripheral plateaus intersect outermost peripheral chimneys of the plurality of chimneys.

6. The holding device of claim 3 or claim 4, wherein the connecting walls extending between the peripheral lower portion and the first and second peripheral plateaus are positioned outside of a receiving region comprising the plurality of chimneys, so as to not be intersected by any chimneys of the plurality of chimneys, with the first and second peripheral plateaus thus extending out past outermost peripheral chimneys of the plurality of chimneys.

7. The holding device of claim 2, wherein the one or more raised plateaus comprise a cross-shaped plateau including:
a first plateau arm and a second plateau arm oriented orthogonal to each other; and
the pair of C-shaped plateaus positioned on opposing ends of the first plateau arm.

8. The holding device of claim 7, wherein the supporting plate comprises a peripheral wall oriented orthogonal to the supporting plate and extending up from the peripheral lower portion, wherein the peripheral wall intersects and is joined to the cross-shaped plateau.

9. The holding device of claim 8, wherein the peripheral wall is positioned to intersect outermost peripheral chimneys of the plurality of chimneys, and wherein the peripheral wall joins to opposing ends of the second plateau arm and intersects the first plateau arm at locations thereof that are aligned with the outermost peripheral chimneys.

10. The holding device of claim 7, wherein the peripheral wall is positioned outside of a receiving region comprising the plurality of chimneys, so as to not be intersected by any chimneys of the plurality of chimneys, and wherein the peripheral wall joins to opposing ends of the second plateau arm to the pair of C-shaped plateaus outside of the receiving region.

11. The holding device of any of claims 7-10, wherein each of the first plateau arm and the second plateau arm includes two bump-out plateaus formed thereon that are further elevated as compared to a remainder of the first plateau arm and the second plateau arm.

12. The holding device of any of claims 1-11, wherein the one or more raised plateaus are elevated relative to the peripheral lower portion by a height that is less than or equal to a height of the plurality of chimneys, such that the one or more raised plateaus intersect at least some chimneys of the plurality of chimneys.

13. The holding device of claim 12, wherein the supporting plate comprises a plurality of reinforcing ribs extending between at least some of the plurality of chimneys, the reinforcing ribs connecting a respective chimney to one or more adjacent chimneys, wherein the reinforcing ribs are orthogonal to the supporting plate and extend up from a bottom end of a respective chimney to the height of the one or more raised plateaus.

14. The holding device of claim 13, wherein the plurality of reinforcing ribs extend between adjacent chimneys encompassed by the center plateau and the pair of C-shaped plateaus, with the reinforcing ribs extending up from the bottom ends of the chimneys to an underside of the center plateau and the pair of C-shaped plateaus.

15. The holding device of claim 13, wherein the plurality of reinforcing ribs extend between adjacent chimneys encompassed by the first plateau arm and the pair of C-shaped plateaus, with the reinforcing ribs extending up from the bottom ends of the chimneys to an underside of the first plateau arm and the pair of C-shaped plateaus.

16. The holding device of any of claims 1-15, wherein respective connecting walls extend between the pair of C-shaped plateaus and the pair of cutouts, providing curved walls at the pair of cutouts that provide finger grips by which a user can grasp the holding device.

17. The holding device of any of claims 1-15, wherein the connecting walls are substantially vertical, extending substantially orthogonally from the lower portion, and having a draft angle of about 0.1 degree to about 4.0 degrees.

18. The holding device of any of claims 1-17, wherein the plurality of chimneys are configured to receive barrels of syringes or cartridges containing a fluid volume of 50 mL or less, preferably from 1-10 mL, and even more preferably from 1-3 mL.

19. The holding device of any of claims 1-18, wherein the peripheral lower portion and the one or more raised plateaus are flat.

20. A method of manufacture for the holding device of claim 1, the method comprising forming the support plate and the plurality of chimneys as a single, integral component.
